# EUROPEAN PATENT SPECIFICATION

(11) **EP 2 018 116 B1**
(45) Date of publication and mention of the grant of the patent: **23.03.2011**
(21) Application number: 06745303.5
(22) Date of filing: 26.04.2006
(51) Int. Cl.: A61B 5/087

(54) **INCENTIVE METHOD FOR THE SPIROMETRY TEST WITH UNIVERSAL CONTROL SYSTEM REGARDLESS OF ANY CHOSEN STIMULATING IMAGE**
ANREIZVERFAHREN FÜR DEN SPIROMETRIE-TEST MIT UNIVERSALKONTROLLSYSTEM UNABHÄNGIG VON EINEM GEWÄHLTEN STIMULATIONSBILD
PROCEDE D'INCITATION POUR TEST DE SPIROMETRIE AVEC UN SYSTEME DE COMMANDE UNIVERSEL QUELQUE SOIT L'IMAGE DE STIMULATION CHOISIE

(43) Date of publication of application: 28.01.2009
(73) Proprietor: MIR SRL Medical International Research, 00155 Roma (IT)
(72) Inventor: BOSCHETTI SACCO, Paolo, I-00155 Roma (IT)
(74) Representative: Sarpi, Maurizio
(86) International application number: PCT/IT2006/000285
(87) International publication number: WO 2007/122653

(56) References cited:
- EP-A- 1 536 364
- WO-A-97/18753
- US-A- 4 114 607
- US-A- 5 333 106
- KOZLOWSKA ET AL: "Spirometry in the pre-school age group" PAEDIATRIC RESPIRATORY REVIEWS, W.B. SAUNDERS LNKD- DOI:10.1016/J.PRRV.2005.09.008, vol. 6, no. 4, 1 December 2005 (2005-12-01), pages 267-272, XP005166859 ISSN: 1526-0542
- GRACCHI V. ET AL: 'Spirometry in young children: should computer-animation programs be used during testing?' EUROPEAN RESPIRATORY JOURNAL, [Online] no. 21, 2003, pages 872 - 875 Retrieved from the Internet: <URL:http://www.ersj.org.uk/content/21/5/87 2.full> [retrieved on 2010-09-06]
- VILOZNI D. ET AL: 'An Interactive Computer-Animated System (SpiroGame) Facilitates Spirometry in Preschool Children' AM. J. RESPIR. CRIT. CARE MED, [Online] vol. 164, no. 12, December 2001, pages 2200 - 2205 Retrieved from the Internet: <URL:http://171.66.122.149/cgi/content/abst ract/164/12/2200>
- INTERNET CITATION: 'Koko Legend Spirometer, Operations Manual', [Online] 2005, Retrieved from the Internet: <URL:http://gracemed.net/uploads/KoKoLegend OperationManual9A.pdf> [retrieved on 2010-09-06]

## Description

Spirometry is a diagnostic test providing the analysis of the respiratory functionality.

The test is carried out by blowing to a spirometer and the correct execution thereof always requires the maximum collaboration of the patient as the result of the test depends on the capability of the patient to breathe out all air of lungs at the maximum possible speed.

If these conditions are not fulfilled, the result of the spirometry is considered not reliable or, as usual in the jargon of physicians, "not acceptable".

The execution of the test can be very difficult for children, elders, handicapped patients or people with psychological troubles.

Therefore, some manufacturers of diagnosis apparatus thought recently to help such measurements by producing incentive systems for the spirometry test which have, however, use and effectiveness limitations.

By way of example, already existing apparatus show the image of a child blowing to the candles on a pie one at a time as the expiration proceeds or a child blowing into a balloon until it burst.

Such systems are conceived so that every stimulating image corresponds to a cartoon control which is firmly related both to the respiration and the imagine itself. Of course, this prevents the image from being replaced by another image.

Kozlowska and Aurora (Spirometry in the pre-school age group, Paediatric respiratory reviews, 2005, issue 6, pages 267-272) disclose an incentive method for the spirometry test, comprising an animation of a bowling ball knocking over skittles, being controlled by an incentive control mechanism based on both flow rate and volume of the exhaled air.

It is clear that in this case the software relating the incentive to the respiration is adapted only to a determined animation and if it is desired to replace the latter with another animation, it is necessary for the control to fit the new one.

It is self-evident that one drawback of the current spirometry incentive systems is the quite limited offer of images or animations made available by the manufacturer of the spirometer.

The main object of the present invention is to overcome the problems of the current spirometry incentive systems by providing a novel incentive control method in which the mechanism relating the cartoon to the respiration is only one and universal, and can be used with a virtually infinite number of incentive images.

This has been accomplished according to the invention by providing a method as defined in claim 1.

A better understanding of the invention will result from the following detailed description with reference to the accompanying drawings that show a preferred embodiment thereof only by way of a not limiting example.

In the drawings:
Figure 1 shows the incentive image in the case of a curtain moving sideways and the relative spirometry diagram before the test;
Figures 2, 3 and 4 similar to fig. 1 relates to the initial and final steps and after the test; and
Figure 5 is a flow chart of the method according to the invention.

As already mentioned above, the existing technology is mainly directed to children so that the incentive systems on the market are exclusively for children's use.

The establishment at the basis of such systems is that children bored, as well-known, by a test carried out in a medical environment can be easily motivated conversely by images stimulating them and suitably related to the respiration.

Therefore, incentives based upon the psychological mechanism of waiting for an event and translated into images related to the test and having an incentive effect have been developed.

The hitherto used technology has the following limits:
- the incentive provides not only the image but also the relative cartoon control software;
- every incentive option has its software and its development cost;
- the software required for every incentive limits necessarily the number thereof produced by the manufacturer and is a strong limitation to the development of new incentives by the physician (or anybody taking care of the patient);

- the limited number of available incentives is destined a priori to determined patients to whom they are referred;
- because of their simple images, the children's incentives that can be found on the market do not have effect to other patients (e.g. elders) that even would need them;
- at last, the greatest limitation among those listed above is that the incentive effect of the system using cartoons is very limited in time with respect to the patient subjected to several spirometry tests. Actually, after one display (e.g. after having blown out one candle on the pie) the surprise effect vanishes as the conclusion is already known;
- considering the limited number of available displays, the conventional incentive systems show the limits of being not effective to the same patient any longer after the latter has understood the mechanism.

### OPERATION OF THE NEW INCENTIVE SYSTEM

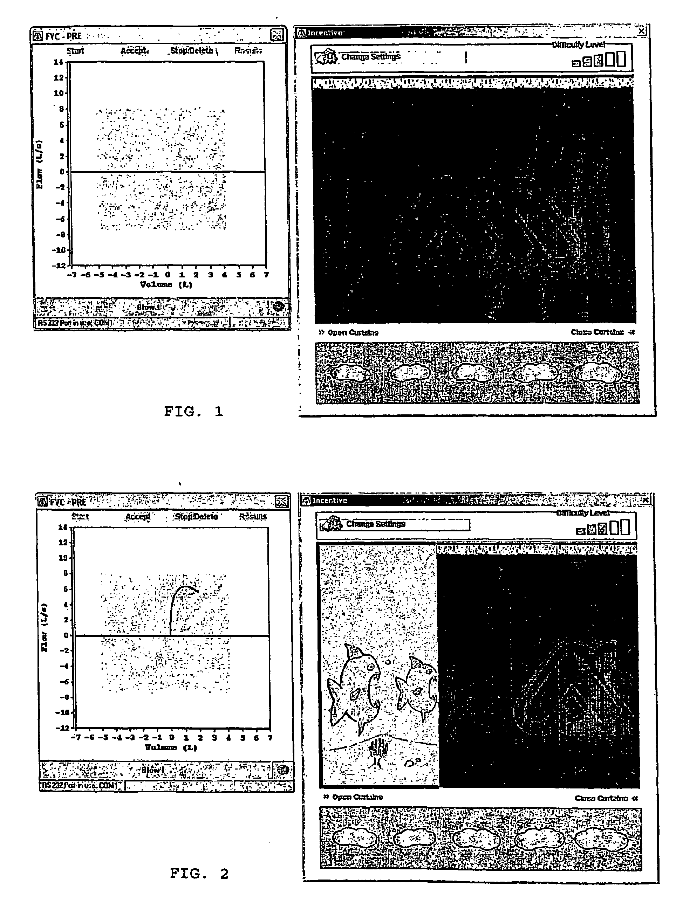

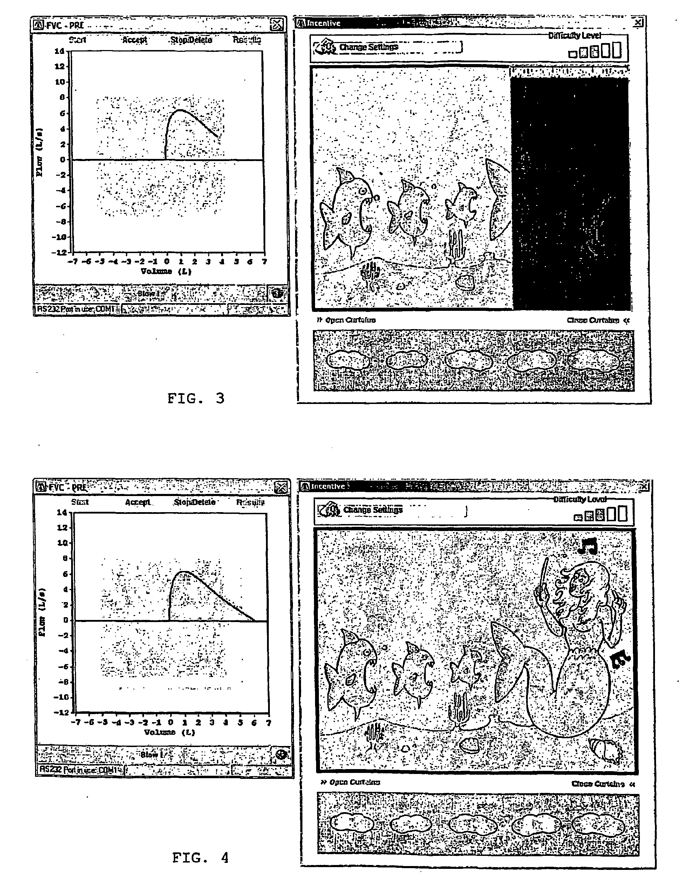

One peculiar feature of the present invention is the "architecture" of the incentive based upon the progressive opening of a curtain (or other alternative mechanism) related to the spirometry test. The curtain in turn hides an image (or animation) which is completely released from and independent of the curtain. The curtain moves as the spirometry test proceeds and unveils gradually the imagine of the "path", i.e. an image having a meaning only if completely unveiled. The incentive effect is then the motivation to blow so that the curtain unveils completely an image which has its completeness and can be interpreted only if completely unveiled (figs. 1 to 4).

Upon carrying out the spirometry test the help of the incentive program is an available option. If the physician opts for it, he/she can further choose between a list of available proposals (fixed or animated images) or can import an image file he/she likes.

Actually, the incentive can be very easily personalised by any image provided that a meaning is given which is incomplete in its first few strokes or however rouses the patient's curiosity to see the whole scene.

Both stationary and dynamic (cartoon) incentives can be used and a series of both types is provided.

The activation of the incentive can either be controlled from time to time or automatically according to the age of the patient.

At last, the incentive system can be used both with a PC connected to the spirometer and directly with the spirometer, of course keeping into account the different memory size and display quality in both cases.

Another peculiar feature of the invention is its calculation algorithm based upon volume and flow rate increments (speed of the expired air) with respect to the "theoretical value" so-called also "awaited value" or "nominal value" of the patient.

The movement of the curtain derives from the combination of these two parameters compared with the theoretical value. Owing to this exclusive algorithm the system is "protected" against non-significant test, for example as the patient blows slowly instead of blowing with the maximum energy.

Furthermore, it is possible to change the level of difficulty to reach the object (correct diagnostic test) by adjusting the "resistance" of the curtain upon opening. This feature makes the incentive very flexible and adapted to several categories of patients.

### ADVANTAGES AND BENEFITS OF THE INVENTION

One advantage of the present invention comes from the new idea of providing an incentive control mechanism which is completely independent of the incentive imagine which makes it unique and universal, i.e. able to control a virtually infinite number of incentive images.

Such mechanism based upon two independent imagines consists, as already mentioned, of the movement of a curtain (similar to the curtain of a theatre) the running of which is controlled via software by means of an algorithm which keeps into account volume and flow rate of the expired air.

The user can put a practically unlimited number of images/cartoons under or, if preferred, behind the curtain in addition to those predefined by the manufacturer without any cost.

The sole feature of the image/cartoon causing the incentive effect is the presence of a "stimulation event" which is unveiled in the end by inducing psychologically the patient to the whole sight. It is easy to understand how any picture or image downloaded from Internet or CD/DVD, or any digital or scanned picture can be used and easily replaced at every spirometry test.

A considerable flexibility and cheapness of use of the mechanism is then reached so that the latter is adapted to a lot of situations as well as keeps always effective and stimulating even if used several times by the same patient.

According to the invention, the sliding of the curtain can take place to the horizontal direction from left to right or vice versa or to the vertical direction from top to bottom and vice versa. For example, the animation of a dropping parachute or a rising balloon (or kite) could be looked at.

Any image dear to a child could be used, for example, an image drawn out of a family album or a cartoon, or a drawing made by the same child which can be easily imported.

According to the present invention an incentive system can be provided which is:
- based upon a control system independent of the incentive image;
- operating together with a mechanism related to the respiration and able to control the progressive display of the image;
- adapted not only to spirometry test but also to any diagnostic test supported by imagines or animations, the result of which is related to the patient's collaboration;
- able to give the display effect by several progressive display systems (curtain, drop-curtain, fading, sliding doors, photographic diaphragm, bellow, shutter, strips, a.s.o.);
- supporting mechanism of moving images to all possible directions (from top to bottom, from bottom to top, from left to right, from right to left, oblique), and fading;
- operating with any incentive image;
- able to change the incentive image easily and without additional cost;
- adapted to different categories of patients;
- not requiring expenses and investments to cover the several categories of patients;
- able to grade the difficulties of the incentive for any patient;
- able to relate the incentive both to volume and flow rate of the expired air;
- able to allow a high number of complete, reliable tests from the diagnostic point of view;
- able to help the patient to understand any mistake made during the spirometry and to keep the motivation of the patient high;
- able to allow a virtually infinite number of test with the same patient keeping the incentive effectiveness unchanged and without additional cost and investments;
- ideal for patient that are unresponsive to spirometry by the conventional systems because of the personalization of the incentive image;
- able to optimise the productivity of the medical staff as well as the number of acceptable spirometries carried out during a determined period of time.

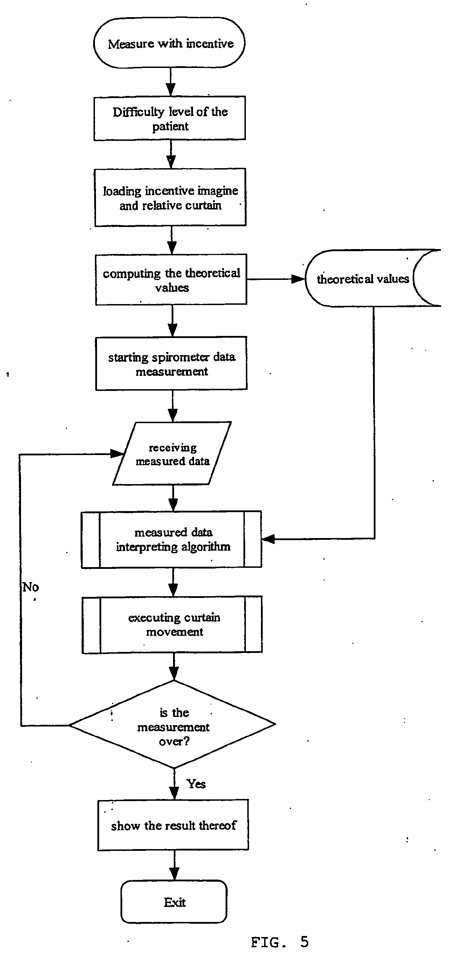

At last, with particular reference to figure 5, the method disclosed above includes the following steps:
1. input of the difficulty level of the patient;
2. loading incentive image and relative curtain;
3. computing the theoretical values;
4. starting spirometer data measurement;
5. receiving measured data;
6. interpreting measured data and comparing them with theoretical data;
7. moving curtain according to the interpretation;
8. is the spirometry over? then show the result thereof, otherwise go back to step 5;
9. ending process after showing the result.

According to the invention, the spirometry test in term of volume and flow rate of the air expired by the patient can also be displayed on a diagram, as shown in figures 1 to 4.

It should be appreciated that upon keeping the inventive concept of the present invention unchanged, a "thick fog" or a "thick smokescreen" which is blown away by the patient during the spirometry test can also be provided as an alternative to the moving curtain.

The present invention has been described and illustrated according to a preferred embodiment thereof, however, it should be understood that those skilled in the art can make technically and functionally equivalent modifications and/or replacements without departing from the scope of the present industrial invention as defined by the claims.

## Claims

1. An incentive method for the spirometry test, comprising the user of a covering image representing a curtain which is progressively opened with an incentive control mechanism related to respiration of the patient controlled via software by an algorithm based on both flow rate and volume of exhaled air; wherein said covering image hides and gradually unveils an inventive image completely independent from both of the covering image and the respiration chosen from a virtually incentive number of fixed or animated images having the function of stimulation event by inducing psychologically the patient to the whole sight wherein the method further comprises a step of choosing said incentive image from a list of availaible fixed or animated images or importing said incentive image as an image file.

2. The method according to the preceding claim, further comprising that said algorithm allows the level of difficulty to be changed in order to carry out a correct diagnostic test by adjusting the effort made by the patient to unveil the incentive image hidden under the covering image so that the incentive is very flexible and adapted to several categories of patients.

3. The method according to claim 1, further comprising that the sliding of the curtain takes place to the horizontal direction from left to right or vice versa or to the vertical direction from top to bottom and vice versa.

4. The method according to claim 1, **characterized by** the following steps:
A. input of the difficulty level of the patient;
B. loading incentive image and relative curtain;
C. computing the theoretical values;
D. starting spirometer data measurement;
E. receiving measured data;
F. interpreting measured data and comparing them with theoretical data;
G. moving said curtain according to the interpretation;
H. if the spirometry over then show the result thereof, otherwise go back to step E;
I. ending process after showing the result.

5. The method according to the preceding claim, that the spirometry test in term of volume and flow rate of the air expired by the patient can also be displayed on a diagram.

## Patentansprüche

1. Incentive-Verfahren für einen Spirometrietest,
umfassend die Verwendung eines Deckbildes entsprechend einem Vorhang, der schrittweise mit einem sich auf die Atmung des Patienten beziehenden Incentive-Kontrollmechanismus geöffnet wird, der über eine Software durch einen Algorithmus basierend auf Durchfluss und Volumen der ausgeatmeten Luft gesteuerten wird,
wobei das Deckbild ein Incentive-Bild verdeckt und graduell sichtbar werden lässt, welches vollkommen unabhängig von sowohl dem verdeckten Bild als auch von der Atmung ist und welches aus einer nahezu unendlichen Anzahl von festen oder animierte Bilder ausgewählt wird und welches mit der Funktion zur Ereignis-Stimulation ausgestattet ist, den Patienten psychologisch zur ganzen Ansicht zu verleiten,
wobei das Incentive-Verfahren ferner einen Schritt zum Auswählen des Incentive-Bildes aus einer Liste von verfügbaren festen oder animierte Bildern oder zum Importieren des Incentive-Bildes aus einer Bild-Datei.

2. Verfahren nach Anspruch 1, ferner aufweisend, dass der Algorithmus erlaubt, einen Schwierigkeitsgrad zu ändern, um eine korrekten diagnostischen Test durch Anpassung eines von dem Patienten getätigten Kraftaufwandes an das Sichtbar werden lassen des versteckten Incentive-Bildes unter dem Deckbild derart durchzuführen, sodass der Anreiz sehr flexibel und an mehrere Kategorien von Patienten angepasst ist.

3. Verfahren nach Anspruch 1, **dadurch gekennzeichnet, dass** das Öffnen des Vorhanges in horizontaler Richtung von links nach rechts oder umgekehrt oder in vertikaler Richtung von oben nach unten oder umgekehrt erfolgt.

4. Verfahren nach Anspruch 1, durch folgende Schritte **gekennzeichnet**:
A) Eingeben des Schwierigkeitsgrades des Patienten;
B) Laden des Incentive-Bildes und des entsprechenden Vorhanges;
C) Berechnen der theoretischen Werte;
D) Starten einer Spirometer-Daten-Messung;
E) Empfangen von gemessenen Daten;
F) Interpretieren der gemessenen Daten und Vergleichen der gemessen Daten mit den theoretischen Werten;
G) Bewegen des Vorhanges entsprechend des Interpretierens;
H) Anzeigen von Ergebnissen, falls der Spirometrietest beendet ist, andernfalls zurück zu Schritt E);
I) Endprozess nach dem Anzeigen der Ergebnisse.

5. Verfahren nach einem der vorhergehenden Ansprüche, **dadurch gekennzeichnet, dass** bei dem Spirometrietest das Volumen und der Durchsatz der vom Patienten ausgeatmeten Luft in einem Diagramm angezeigt werden kann.

## Revendications

1. Procédé d'incitation pour le test de spirométrie, comprenant l'utilisation d'une image de recouvrement représentant un rideau qui est ouvert progressivement par un mécanisme de commande d'incitation en relation avec la respiration du patient commandé par l'intermédiaire d'un logiciel par un algorithme basé à la fois sur le débit et le volume d'air exhalé, dans lequel ladite image de recouvrement cache et dévoile graduellement une image d'incitation complètement indépendante à la fois de l'image de recouvrement et de la respiration choisie parmi un nombre pratiquement infini d'images fixes ou animées ayant la fonction d'événement de stimulation en incitant psychologiquement le patient à découvrir la vue entière, dans lequel le procédé comprend en outre une étape consistant à choisir ladite image d'incitation dans une liste d'images fixes ou animées disponibles ou à importer ladite image d'incitation en tant que fichier d'image.

2. Procédé selon la revendication précédente, consistant en outre en ce que ledit algorithme permet de modifier le niveau de difficulté afin d'effectuer un test de diagnostic correct en ajustant l'effort effectué par le patient pour dévoiler l'image d'incitation cachée sous l'image de recouvrement de sorte que l'incitation soit très flexible et adaptée à plusieurs catégories de patients.

3. Procédé selon la revendication 1, consistant en outre en ce que le coulissement du rideau a lieu dans la direction horizontale de gauche à droite ou vice versa ou dans la direction verticale du haut vers le bas et vice versa.

4. Procédé selon la revendication 1, **caractérisé par** les étapes suivantes :
A. entrer le niveau de difficulté du patient ;
B. charger une image d'incitation et un rideau relatif ;
C. calculer les valeurs théoriques ;
D. débuter la mesure de données de spirométrie ;
E. recevoir les données mesurées ;
F. interpréter les données mesurées et les comparer aux données théoriques ;
G. déplacer ledit rideau conformément à l'interprétation ;
H. si la spirométrie est terminée, présenter alors le résultat de celle-ci, autrement retourner à l'étape E ;
I. terminer le processus après la présentation du résultat.

5. Procédé selon la revendication précédente, **caractérisé en ce que** le test de spirométrie en termes de volume et de débit de l'air expiré par le patient peut également être affiché sur un diagramme.
